(19) Europäisches Patentamt
European Patent Office
Office européen des brevets

(11) **EP 4 104 991 A1**

(12) **EUROPEAN PATENT APPLICATION**
published in accordance with Art. 153(4) EPC

(43) Date of publication:
**21.12.2022 Bulletin 2022/51**

(21) Application number: **21792721.9**

(22) Date of filing: **19.04.2021**

(51) International Patent Classification (IPC):
**B29B 11/14** (2006.01)　　**B29C 49/06** (2006.01)
**B29C 49/42** (2006.01)　　**A61L 2/08** (2006.01)

(52) Cooperative Patent Classification (CPC):
**A61L 2/08; B29B 11/14; B29C 49/06; B29C 49/42**

(86) International application number:
**PCT/JP2021/015934**

(87) International publication number:
**WO 2021/215413 (28.10.2021 Gazette 2021/43)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **22.04.2020　JP 2020075913**

(71) Applicant: **Mitsubishi Heavy Industries Machinery
Systems, Ltd.
Kobe-shi, Hyogo 652-8585 (JP)**

(72) Inventors:
• **SUGIYAMA, Shigehiro
Tokyo 100-8332 (JP)**
• **UEDA, Atsushi
Kobe-shi, Hyogo 652-8585 (JP)**

(74) Representative: **Hoffmann Eitle
Patent- und Rechtsanwälte PartmbB
Arabellastraße 30
81925 München (DE)**

(54) **PREFORM, STERILIZATION DEVICE, AND STERILIZATION METHOD**

(57) Provided is a preform (100) comprising: a plug section (10) that is formed in a cylindrical shape along an axis (X) and has one end open; a trunk section (20) that is connected to the plug section (10); and a base section (30) that is connected to the trunk section (20) and has one end along the axis (X) closed. The trunk section (20) includes: a linear section (21) that is connected to the plug section (10) and has an internal diameter (D1) which is constant at any position along the axis (X); and an inclined section (22) one end of which is connected to the linear section (21) and another end of which is connected to the base section (30), the internal diameter (D2) of the inclined section becoming gradually less at a constant gradient along the axis (X) from the linear section (21) toward the base section (30). The inclined section (22) is formed so that the angle of inclination ($\theta$) of an inner peripheral surface (22a) thereof from the axis (X) is 12°-22°.

FIG. 1

## Description

Technical Field

The present disclosure relates to a preform, a sterilization device, and a sterilization method.

Background Art

In the related art, an electron beam sterilization device that irradiates a preform of a container before blow molding with an electron beam to sterilize the preform is known (for example, refer to PTL 1). The electron beam sterilization device disclosed in PTL 1 inserts an electron beam irradiation nozzle in a fixed shaft state into the preform by using a raising-lowering device to raise and lower the preform with respect to the electron beam irradiation nozzle. The electron beam sterilization device disclosed in PTL 1 performs a sterilization treatment on an inner surface of the preform by irradiating the inner surface of the preform with the electron beam in a state where the electron beam irradiation nozzle is inserted inside the preform.

Citation List

Patent Literature

[PTL 1] Japanese Patent No. 6091373

Summary of Invention

Technical Problem

However, the electron beam sterilization device disclosed in the patent literature requires the raising-lowering device for raising and lowering the preform with respect to the electron beam irradiation nozzle. Therefore, manufacturing costs of the electron beam sterilization device increase. In addition, it takes time to perform an operation for raising and lowering the preform with respect to the electron beam irradiation nozzle. Consequently, a time required for performing the sterilization treatment on the preform is lengthened.

In order to avoid an increase in the manufacturing costs of the electron beam sterilization device or the time required for performing the sterilization treatment, it is conceivable to apply a method for irradiating an inside of the preform with the electron beam while transporting the preform. However, when an absorbed radiation dose of the electron beam is not sufficient in a base section of the preform where a distance from an irradiation source of the electron beam is long, there is a possibility that the sterilization treatment may not be sufficiently performed. On the other hand, when acceleration energy of the electron beam is excessively high to obtain the sufficient absorbed radiation dose of the electron beam in the base section of the preform, the absorbed radiation dose of the electron beam is excessively large at a position where the distance from the irradiation source of the electron beam is short. Therefore, there is a possibility that the preform may be deformed or melted.

The present disclosure is made in view of the above-described circumstances, and an object of the present disclosure is to provide a preform, a sterilization device, and a sterilization method which can prevent an absorbed radiation dose of an electron beam from being excessively large on a plug section side of the preform while the absorbed radiation dose of the electron beam is sufficiently secured on a base section side of the preform, when a sterilization treatment is performed on the preform by irradiating the preform with an electron beam.

Solution to Problem

According to an aspect of the present disclosure, there is provided a preform including a plug section formed in a cylindrical shape along an axis and having one end open, a trunk section formed in a cylindrical shape along the axis and connected to the plug section, and a base section connected to the trunk section and having one end closed along the axis. The trunk section has a linear section connected to the plug section and whose first inner diameter in a radial direction orthogonal to the axis is constant at each position along the axis, and an inclined section of which one end along the axis is connected to the linear section and the other end along the axis is connected to the base section, and whose second inner diameter in the radial direction gradually decreases at a constant gradient from the linear section toward the base section along the axis. The inclined section is formed so that an inclination angle of an inner peripheral surface with respect to the axis is 12 degrees or larger and 22 degrees or smaller.

According to another aspect of the present disclosure, there is provided a sterilization method for sterilizing a preform in which the preform includes a plug section formed in a cylindrical shape along an axis and having one end open, a trunk section formed in a cylindrical shape along the axis and connected to the plug section, and a base section connected to the trunk section and having one end closed along the axis, the trunk section has a linear section connected to the plug section and whose first inner diameter in a radial direction orthogonal to the axis is constant at each position along the axis, and an inclined section of which one end along the axis is connected to the linear section and the other end along the axis is connected to the base section, and whose second inner diameter in the radial direction gradually decreases at a constant gradient from the linear section toward the base section along the axis, and the inclined section is formed so that an inclination angle of an inner peripheral surface with respect to the axis is 12 degrees or larger and 22 degrees or smaller. The sterilization method includes a transport step of transporting the preform along a transport direction intersecting with the axis, and an irradiation step of irradiating the plug section of the preform with an electron beam in a direction along the axis in a transport path through which the preform is transported by performing the transport step.

Advantageous Effects of Invention

According to the present disclosure, it is possible to provide a preform, a sterilization device, and a sterilization method which can prevent an absorbed radiation dose of an electron beam from being excessively large on a plug section side of the preform while the absorbed radiation dose of the electron beam is sufficiently secured on a base section side of the preform, when a sterilization treatment is performed on the preform by irradiating the preform with an electron beam.

Brief Description of Drawings

Fig. 1 is a longitudinal sectional view of a preform according to an embodiment of the present disclosure.
Fig. 2 is a view illustrating a state where the preform is irradiated with an electron beam from an irradiation unit.
Fig. 3 is a graph illustrating a relationship between a distance from an end portion of the preform and an absorbed radiation dose of the electron beam.
Fig. 4 is a view illustrating a sterilization device according to an embodiment of the present disclosure.
Description of Embodiments

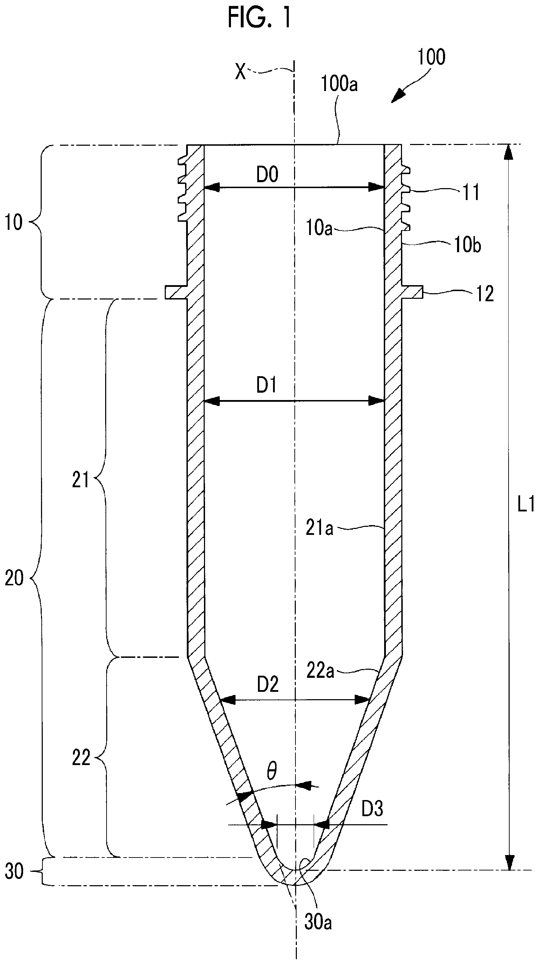

Hereinafter, an embodiment of a preform, a sterilization device, and a sterilization method according to the present disclosure will be described with reference to the drawings. Fig. 1 is a longitudinal sectional view of a preform 100 according to an embodiment of the present disclosure.
The preform 100 of the present embodiment is a tubular member having a base section integrally molded from a thermoplastic resin material serving as a primary material, and is a member for manufacturing a plastic bottle through blow molding. For example, the thermoplastic resin material used for the preform 100 is polyethylene (PE), polypropylene (PP), polyethylene terephthalate (PET), polyethylene naphthalate (PEN), and polycarbonate (PC).
As illustrated in Fig. 1, the preform 100 of the present embodiment includes a plug section 10, a trunk section 20, and a base section 30. The plug section 10, the trunk section 20, and the base section 30 are integrally molded from the thermoplastic resin material. The preform 100 has a shape extending along an axis X, in which one end is open on the plug section 10 side and the other end is closed on the base section 30 side.
An inner diameter D0 of an inner peripheral surface 10a of the plug section 10 is constant at each position along the axis X. A length along the axis X from an end portion 100a on the plug section 10 side of the preform 100 to a position where the inner peripheral surface 30a of the base section 30 and the axis X intersect with each other is defined as L1. For example, in a case of the preform 100 used for a plastic bottle having a volume of 600 mL or smaller, the length L1 is set to 90 mm or longer and 110 mm or shorter.
The plug section 10 is formed in a cylindrical shape along the axis X, and has a shape in which one end is open in the end portion 100a. A male screw 11 spirally extending around the axis X is formed on an outer peripheral surface 10b of the plug section 10. The male screw 11 is used to attach a cap (not illustrated) to a plastic bottle manufactured from the preform 100.
An annular support ring 12 protruding outward from the outer peripheral surface 10b in a radial direction orthogonal to the axis X is provided in an end portion of the plug section 10 on the base section 30 side. The support ring 12 is a member for holding a detachment ring (not illustrated) which is detached from a cap when the plastic bottle is opened.
The trunk section 20 is formed in a cylindrical shape along the axis X, one end is connected to the plug section 10, and the other end is connected to the base section 30. The trunk section 20 has a linear section 21 connected to the plug section 10 and an inclined section 22 connected to the linear section 21 and the base section 30.
The linear section 21 is a cylindrical member in which one end along the axis X is connected to the plug section 10 and the other end along the axis X is connected to the inclined section 22. An inner diameter (first inner diameter) D1 of an inner peripheral surface 21a of the linear section 21 is constant at each position along the axis X. The inner diameter D1 of the inner peripheral surface 21a of the linear section 21 is the same as the inner diameter D0 of the inner peripheral surface 10a of the plug section 10. For example, the inner diameter D1 is set to 20 mm or larger and 35 mm or smaller.
The inclined section 22 is a hollow conical member in which one end along the axis X is connected to the linear section 21 and the other end along the axis X is connected to the base section 30. The inner diameter (second inner diameter) D2 of the inner peripheral surface 22a of the inclined section 22 gradually decreases with a constant gradient from the linear section 21 toward the base section 30 along the axis X. An inner diameter D2 of an inner peripheral surface 22a of the inclined section 22 gradually decreases from the inner diameter D1 of the linear section 21 to the inner diameter D3 of the upper end portion of the base section 30 as the inclined section 22 is closer to the base section 30 from the linear section 21.
As illustrated in Fig. 1, in the inclined section 22, an inclination angle of the inner peripheral surface 22a with respect to the axis X is defined as θ. In the present embodiment, the inclination angle θ is set to satisfy Equation (1) below.

$$12° \leq \theta \leq 22° \quad (1)$$

In addition, it is more desirable that the inclination angle θ is set to satisfy Equation (2) below.

$$14° \leq \theta \leq 20° \quad (2)$$

The base section 30 has a shape connected to the trunk section 20 and having one end closed along the axis X. The base section 30 is a section with which a stretch rod (not illustrated) comes into contact when stretch blow molding is performed.

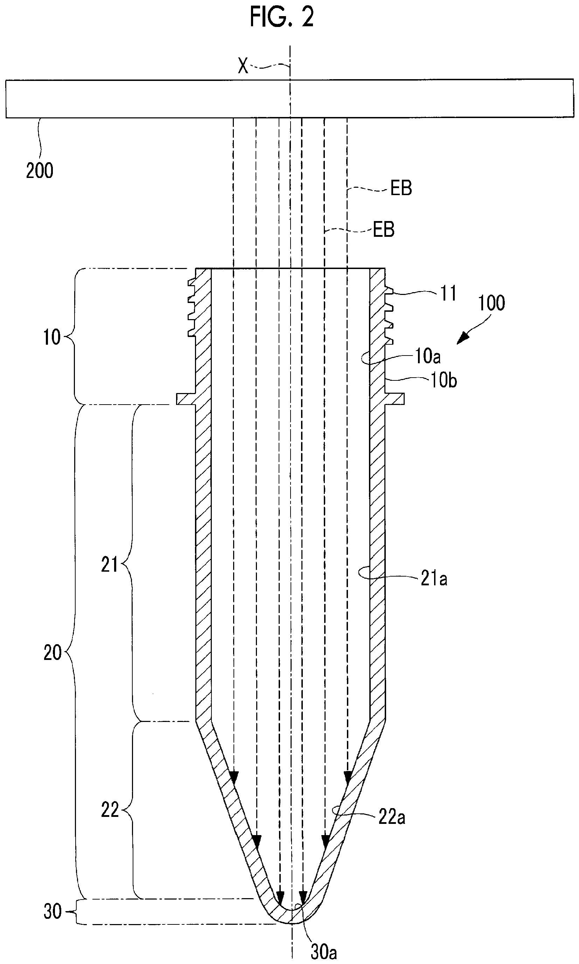

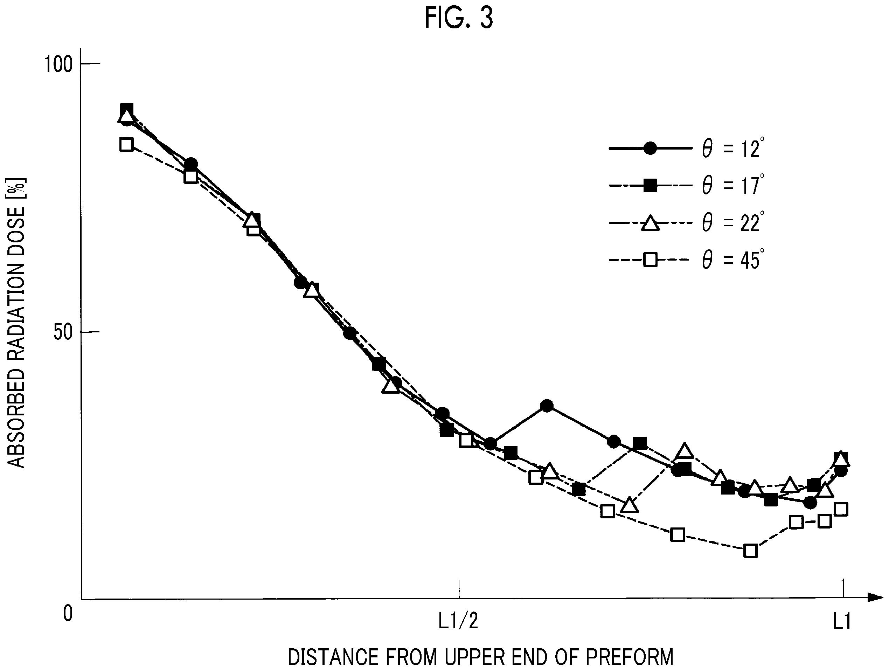

Next, referring to Figs. 2 and 3, a reason that the preform 100 of the present embodiment adopts a shape having the inclined section 22 will be described. Fig. 2 is a view illustrating a state where the preform 100 is irradiated with an electron beam EB from an irradiation unit 200. Fig. 3 is a graph illustrating a relationship between a distance from the end portion 100a of the preform 100 and an absorbed radiation dose.

As will be described later, in the present embodiment, in order to avoid an increase in manufacturing costs of a sterilization device 400 and a time required for a sterilization treatment, a sterilization method is adopted as follows. While the preform 100 is transported, an inside of the preform 100 is irradiated with the electron beam. This method is advantageously used in terms of the manufacturing costs, since the method does not require a raising-lowering device of the preform 100, compared to a method of inserting an electron beam irradiation nozzle inside the preform 100.

On the other hand, the preform 100 is not irradiated with the electron beam inside the preform 100, and is irradiated with the electron beam outside the preform 100. Accordingly, when the absorbed radiation dose of the electron beam is not sufficient in the base section 30 of the preform 100 where a distance from an irradiation source of the electron beam is long, there is a possibility that a sterilization treatment may not be sufficiently performed. Furthermore, when acceleration energy of the electron beam excessively increases to obtain the sufficient absorbed radiation dose of the electron beam in the base section 30 of the preform 100, the absorbed radiation dose of the electron beam excessively increases at a position where a distance from the irradiation source of the electron beam is short. Consequently, there is a possibility that the preform 100 may be deformed or melted.

As illustrated in Fig. 2, in the present embodiment, the irradiation unit 200 that irradiates the preform 100 with the electron beam EB is used to sterilize the inner peripheral surface 10a, the inner peripheral surface 21a, the inner peripheral surface 22a, and the inner peripheral surface 30a, which are the inner peripheral surfaces of the preform 100. The irradiation unit 200 is a device that irradiates the plug section 10 of the preform 100 along the axis X with the electron beam EB.

As illustrated in Fig. 2, in the preform 100 of the present embodiment, the inner diameter D0 of the plug section 10 and the inner diameter D1 of the linear section 21 connected to the plug section 10 are constant at each position along the axis X. Therefore, when the preform 100 is irradiated with the electron beam EB along the axis X, the absorbed radiation dose of the electron beam EB in the plug section 10 and the linear section 21 relatively decreases.

On the other hand, in the preform 100 of the present embodiment, the inner diameter D2 of the inclined section 22 connected to the linear section 21 gradually decreases toward the base section 30 at a constant gradient from the inner diameter D1 to the inner diameter D3. Accordingly, the absorbed radiation dose of the electron beam EB in the inclined section 22 relatively increases when the preform 100 is irradiated the electron beam EB along the axis X. Therefore, while the absorbed radiation dose of the electron beam EB on the base section 30 side of the preform 100 can be sufficiently secured, the absorbed radiation dose of the electron beam EB on the plug section 10 side of the preform 100 can be prevented from being excessively large.

In addition, according to the preform 100 of the present embodiment, the inclined section 22 is formed so that the inclination angle θ of the inner peripheral surface 22a with respect to the axis X is 12 degrees or larger and 22 degrees or smaller. As the inclination angle θ increases, the absorbed radiation dose per unit area on the base section 30 side increases. However, a region of the inclined section 22 is narrowed, and the absorbed radiation dose of the whole inclined section decreases. In addition, as the inclination angle θ decreases, the region of the inclined section 22 is widened. However, the absorbed radiation dose per unit area on the base section 30 side decreases.

As a result of studying the inclination angle of the inclined section while adjusting the inclination angle of the inclined section, the inventors have found the followings. When the inclined section 22 is formed so that the inclination angle is 12 degrees or larger and 22 degrees or smaller, while the absorbed radiation dose of the electron beam EB can be sufficiently secured on the base section 30 side of the preform 100, the absorbed radiation dose of the electron beam EB can be prevented from being excessively large on the plug section 10 side of the preform 100.

Fig. 3 is a graph illustrating a relationship between a distance from the end portion 100a of the preform 100 and the absorbed radiation dose of the electron beam. A horizontal axis illustrated in Fig. 3 indicates a distance in an X-axis direction from the end portion 100a of the inner peripheral surface of the preform 100 when a position of the end portion 100a of the preform 100 is set to 0. A maximum value of the distance in the X-axis direction from the end portion 100a is defined as L1.

A vertical axis illustrated in Fig. 3 indicates a ratio of the absorbed radiation dose of the electron beam EB per unit time and per unit area of the inner peripheral surface of the preform 100 when a predetermined reference value is set to 100%. A plot illustrated in Fig. 3 indicates a ratio of the absorbed radiation dose obtained when the inclination angles θ are set to 12°, 17°, 22°, and 45°. An example in which the inclination angles θ are set to 12°, 17°, and 22° is an example of the present embodiment which is included in a range of Equation (1) described above. On the other hand, an example in which the inclination angle θ is set to 45° is a comparative example of the present embodiment which is not included in the range of Equation (1) described above.

As illustrated in Fig. 3, in the example where the inclination angle θ is set to 45°, the absorbed radiation dose greatly decreases after the distance in the X-axis direction from the end portion 100a of the inner peripheral surface of the preform 100 exceeds L1 / 2, and the absorbed radiation dose slightly increases immediately before the distance in the X-axis direction is close to L1. On the other hand, in the example in which the inclination angles θ are 12°, 17°, and 22°, after the distance in the X-axis direction from the end portion 100a of the inner peripheral surface of the preform 100 exceeds L1 / 2, the absorbed radiation dose increases by switching the linear section 21 to the inclined section 22.

In the example where the inclination angles θ are set to 12°, 17°, and 22°, the absorbed radiation dose decreases as the distance in the X-axis direction is closer to L1 after the absorbed radiation dose increases. However, the absorbed radiation dose at each position from a position where the distance in the X-axis direction is L1 / 2 to the position where the distance is L1 maintains a state where the absorbed radiation dose is larger than that of the comparative example in which the inclination angle θ is set to 45°. Based on the above-described result, the inventors set the inclination angle θ within the range of Equation (1) described above.

In the present embodiment, the length L1 along the axis X from the end portion 100a on the plug section 10 side of the preform 100 to a position where the inner peripheral surface 30a of the base section 30 and the axis X intersect with each other is set to satisfy Equation (3) below.

$$0.5 \cdot D1 \leq L1 \leq 3.0 \cdot D1 \quad (3)$$

In addition, it is more desirable that the length L1 is set to satisfy Equation (4) below.

$$1.0 \cdot D1 \leq L1 \leq 2.6 \cdot D1 \quad (4)$$

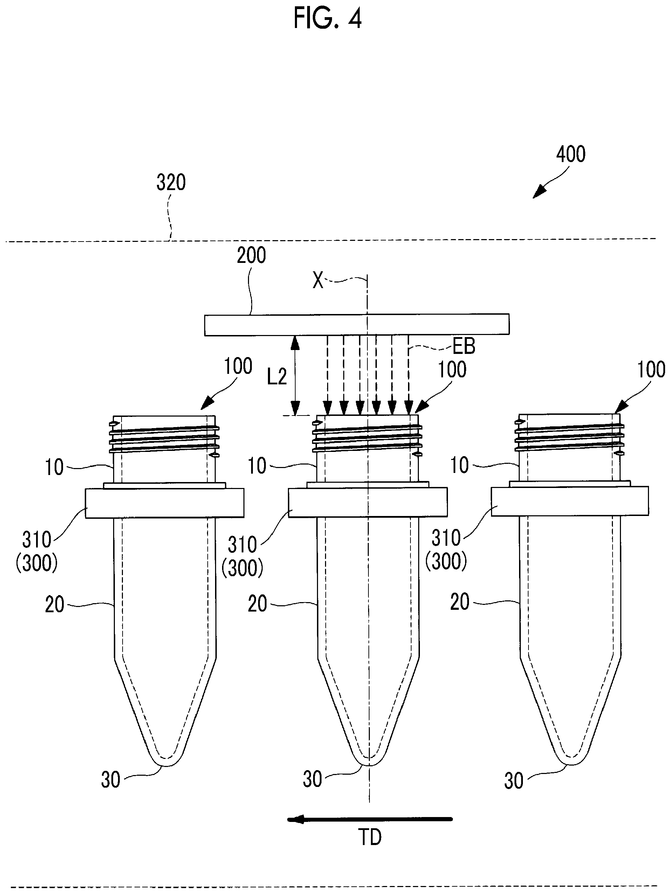

Next, the sterilization device 400 of the present embodiment will be described with reference to Fig. 4. Fig. 4 is a view illustrating the sterilization device 400 according to the present embodiment. As illustrated in Fig. 4, the sterilization device 400 includes the irradiation unit 200 and the transport unit 300. An example illustrated in Fig. 4 indicates a state where the transport unit 300 transports three preforms 100. However, the transport unit 300 can transport any desired number of the preforms 100, for example, three or more preforms 100.

The irradiation unit 200 is a device that irradiates the plug section 10 of the preform 100 with the electron beam EB in the direction along the axis X. The irradiation unit 200 is disposed in the transport path 320 through which the transport unit 300 transports the preform 100.

The irradiation unit 200 irradiates the preform 100 with the electron beam EB so that the electron beam EB reaches the base section 30 from the outside of the preform 100 via the plug section 10. A length L2 along the axis X from a position where the irradiation unit 200 of the present embodiment irradiates the preform 100 with the electron beam EB to the plug section 10 of the preform 100 is set to approximately 20 mm, for example.

For example, the acceleration energy of the electron beam EB used for irradiation from the irradiation unit 200 is set to 120 eV or higher and 180 eV or lower. For example, it is desirable to set the acceleration energy so that a minimum value of the absorbed radiation dose of the electron beam EB in the inner peripheral surface of the preform 100 is approximately 15 kGy.

The transport unit 300 is a device that transports a plurality of the preforms 100 along a transport direction TD orthogonal to (intersecting with) the axis X. The transport unit 300 includes a plurality of holding portions 310 for holding the plurality of preforms 100. The transport unit 300 transports the preform 100 to pass below the irradiation unit 200 disposed in the transport path 320 by moving the holding portion 310 along the transport direction TD at a constant speed. For example, a transport speed at which the transport unit 300 transports the preform 100 along the transport direction TD is set to 300 mm/sec or higher and 500 mm/sec or lower.

The sterilization device 400 of the present embodiment performs a sterilization treatment on the preform 100 as follows.

First, the preform 100 is installed to be held by the holding portion 310 of the transport unit 300.

Second, in a state where the preform 100 is held by the holding portion 310, the preform 100 is transported along the transport direction TD by the transport unit 300 (transport step).

Third, in a state where the preform 100 is transported along the transport direction TD by the transport unit 300, the preform 100 is irradiated with the electron beam EB from the irradiation unit 200, and the electron beam is caused to reach the inner peripheral surface of the preform 100 passing below the irradiation unit 200 (irradiation step).

For example, the preform (100) described in each of the above-described embodiments can be understood as follows.

According to the present disclosure, there is provided the preform including the plug section (10) formed in a cylindrical shape along the axis (X) and having one end open, the trunk section (20) formed in a cylindrical shape along the axis (X) and connected to the plug section (10), and the base section (30) connected to the trunk section and having one end closed along the axis. The trunk section has the linear section (21) connected to the plug section and whose first inner diameter (D1) in the radial direction orthogonal to the axis is constant at each position along the axis, and the inclined section (22) of which one end along the axis is connected to the linear section and the other end along the axis is connected to the base section, and whose second inner diameter (D2) in the radial direction gradually decreases at a constant gradient from the linear section toward the base section along the axis. The inclined section is formed so that the inclination angle of the inner peripheral surface with respect to the axis is 12 degrees or larger and 22 degrees or smaller.

According to the preform of the present disclosure, the trunk section connected to the plug section has the linear section connected to the plug section and whose first inner diameter in the radial direction is constant at each position along the axis, and the inclined section connected to the linear section and whose second inner diameter in the radial direction gradually decreases at the constant gradient from the linear section toward the base section.

The first inner diameter of the linear section connected to the plug section is constant at each position along the axis. Therefore, the absorbed radiation dose of the linear section relatively decreases when the preform is irradiated with the electron beam along the axis. On the other hand, the second inner diameter of the inclined section connected to the linear section gradually decreases at the constant gradient toward the base section. Therefore, the absorbed radiation dose of the inclined section relatively increases when the preform is irradiated with the electron beam along the axis. Therefore, while the absorbed radiation dose of the electron beam on the base section side of the preform can be sufficiently secured, the absorbed radiation dose of the electron beam on the plug section side of the preform can be prevented from being excessively large.

In addition, according to the preform of the present disclosure, the inclined section is formed so that the inclination angle of the inner peripheral surface with respect to the axis is 12 degrees or larger and 22 degrees or smaller. As the inclination angle increases, the absorbed radiation dose per unit area on the base section side increases. However, the region of the inclined section is narrowed, and the absorbed radiation dose of the whole inclined section decreases. In addition, as the inclination angle decreases, the region of the inclined section is widened. However, the absorbed radiation dose per unit area on the base section side decreases. As a result of studying the inclination angle of the inclined section while adjusting the inclination angle of the inclined section, the inventors have found the followings. When the inclined section is formed so that the inclination angle is 12 degrees or larger and 22 degrees or smaller, while the absorbed radiation dose of the electron beam can be sufficiently secured on the base section side of the preform, the absorbed radiation dose of the electron beam can be prevented from being excessively large on the plug section side of the preform.

In the preform according to the present disclosure, the length from the end portion of the plug section (10) along the axis (X) to the base section (30) may be equal to or shorter than three times the first inner diameter (D1).

Since the length from the end portion of the plug section to the base section is equal to or shorter than three times the first inner diameter of the linear section, the distance from the irradiation source of the electron beam to the base section is sufficiently short with respect to the first inner diameter of the linear section. Therefore, the distance from the irradiation source of the electron beam to the base section is lengthened with respect to the first inner diameter, and the absorbed radiation dose of the electron beam on the base section side can be prevented from being excessively reduced.

For example, the sterilization device (300) in each of the above-described embodiments can be understood as follows.

According to the present disclosure, there is provided the sterilization device including the transport unit (300) that transports the above-described preform along the transport direction (TD) intersecting with the axis (X), and the irradiation unit (200) disposed in the transport path (320) through which the transport unit (300) transports the preform, and irradiating the plug section of the preform with the electron beam in the direction along the axis.

According to the sterilization device of the present disclosure, while the absorbed radiation dose of the electron beam can be sufficiently secured on the base section side of the preform, the absorbed radiation dose of the electron beam can be prevented from being excessively large on the plug section side of the preform. In addition, it is not necessary to insert the irradiation source of the electron beam into the preform. Therefore, manufacturing costs of the sterilization device can be reduced.

For example, the sterilization method in each of the above-described embodiments can be understood as follows.

According to the present disclosure, there is provided the sterilization method for sterilizing the preform, in which the preform includes the plug section formed in a cylindrical shape along the axis and having one end open, the trunk section formed in a cylindrical shape along the axis and connected to the plug section, and the base section connected to the trunk section and having one end closed along the axis. The trunk section has the linear section connected to the plug section and whose first inner diameter in the radial direction orthogonal to the axis is constant at each position along the axis, and the inclined section of which one end along the axis is connected to the linear section and the other end along the axis is connected to the base section, and whose second inner diameter in the radial direction gradually decreases at a constant gradient from the linear section toward the base section along the axis. The inclined section is formed so that an inclination angle of an inner peripheral surface with respect to the axis is 12 degrees or larger and 22 degrees or smaller. The sterilization method includes the transport step of transporting the preform along a transport direction intersecting with the axis, and the irradiation step of irradiating the plug section of the preform with an electron beam in a direction along the axis in a transport path through which the preform is transported by performing the transport step.

According to the sterilization method of the present disclosure, while the absorbed radiation dose of the electron beam can be sufficiently secured on the base section side of the preform, the absorbed radiation dose of the electron beam can be prevented from being excessively large on the plug section side of the preform. In addition, it is not necessary to insert the irradiation source of the electron beam into the preform. Therefore, a sterilization treatment can be performed on the preform by using a relatively easy method.

Reference Signs List

| | |
|---|---|
| 10: | Plug section |
| 20: | Trunk section |
| 21: | Linear section |
| 21a: | Inner peripheral surface |
| 22: | Inclined section |
| 22a: | Inner peripheral surface |
| 30: | Base section |
| 30a: | Inner peripheral surface |
| 100: | Preform |
| 100a: | End portion |
| 200: | Irradiation unit |
| 300: | Transport unit |
| 320: | Transport path |
| 400: | Sterilization device |
| EB: | Electron beam |
| TD: | Transport direction |
| X: | Axis |
| θ: | Inclination angle |

## Claims

1. A preform comprising:

   a plug section formed in a cylindrical shape along an axis and having one end open;
   a trunk section formed in a cylindrical shape along the axis and connected to the plug section; and
   a base section connected to the trunk section and having one end closed along the axis, wherein the trunk section has

   a linear section connected to the plug section and whose first inner diameter in a radial direction orthogonal to the axis is constant at each position along the axis, and an inclined section of which one end along the axis is connected to the linear section and the other end along the axis is connected to the base section, and whose second inner diameter in the radial direction gradually decreases at a constant gradient from the linear section toward the base section along the axis, and

   the inclined section is formed so that an inclination angle of an inner peripheral surface with respect to the axis is 12 degrees or larger and 22 degrees or smaller.

2. The preform according to Claim 1, wherein a length from an end portion of the plug section to the base section along the axis is equal to or shorter than 3 times the first inner diameter.

3. A sterilization device comprising:

   a transport unit that transports the preform according to Claim 1 or 2 along a transport direction intersecting with the axis; and
   an irradiation unit disposed in a transport path through which the transport unit transports the preform, and irradiating the plug section of the preform with an electron beam in a direction along the axis.

4. A sterilization method for sterilizing a preform, in which the preform includes

   a plug section formed in a cylindrical shape along an axis and having one end open,
   a trunk section formed in a cylindrical shape along the axis and connected to the plug section, and
   a base section connected to the trunk section and having one end closed along the axis,

the trunk section has

a linear section connected to the plug section and whose first inner diameter in a radial direction orthogonal to the axis is constant at each position along the axis, and

an inclined section of which one end along the axis is connected to the plug section and the other end along the axis is connected to the base section, and whose second inner diameter in the radial direction gradually decreases at a constant gradient from the plug section toward the base section along the axis, and

the inclined section is formed so that an inclination angle of an inner peripheral surface with respect to the axis is 12 degrees or larger and 22 degrees or smaller,

the sterilization method comprising:

a transport step of transporting the preform along a transport direction intersecting with the axis; and

an irradiation step of irradiating the plug section of the preform with an electron beam in a direction along the axis in a transport path through which the preform is transported by performing the transport step.

## FIG. 1

# FIG. 2

FIG. 3

DISTANCE FROM UPPER END OF PREFORM

EP 4 104 991 A1

# FIG. 4

## INTERNATIONAL SEARCH REPORT

International application No.

**PCT/JP2021/015934**

### A. CLASSIFICATION OF SUBJECT MATTER

**B29B 11/14**(2006.01)i; **B29C 49/06**(2006.01)i; **B29C 49/42**(2006.01)i; **A61L 2/08**(2006.01)i
FI: B29B11/14; B29C49/42; B29C49/06; A61L2/08 108

According to International Patent Classification (IPC) or to both national classification and IPC

### B. FIELDS SEARCHED

Minimum documentation searched (classification system followed by classification symbols)

B29B11/14; B29C49/06; B29C49/42; A61L2/08

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2005-067002 A (TOYO SEIKAN KAISHA, LTD.) 17 March 2005 (2005-03-17) paragraphs [0017]-[0024], fig. 2-3 | 1-2 |
| Y | | 3-4 |
| X | JP 2011-000815 A (AOKI TECHNICAL LABORATORY INC.) 06 January 2011 (2011-01-06) claims, paragraphs [0018]-[0023], fig. 2, 4 | 1-2 |
| Y | | 3-4 |
| Y | JP 2017-209136 A (SHIBUYA KOGYO CO., LTD.) 30 November 2017 (2017-11-30) claims | 3-4 |
| A | JP 2001-225814 A (TOYO SEIKAN KAISHA, LTD.) 21 August 2001 (2001-08-21) paragraphs [0024]-[0043] | 1-4 |
| A | JP 2013-226709 A (TOPPAN PRINTING CO., LTD.) 07 November 2013 (2013-11-07) entire text, all drawings | 1-4 |

☐ Further documents are listed in the continuation of Box C.   ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **22 June 2021** | **06 July 2021** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**
Information on patent family members

International application No.

**PCT/JP2021/015934**

| Patent document cited in search report | Publication date (day/month/year) | Patent family member(s) | Publication date (day/month/year) |
|---|---|---|---|
| JP     2005-067002     A | 17 March 2005 | (Family: none) | |
| JP     2011-000815     A | 06 January 2011 | US     2010/0323136     A1 paragraphs [0031]-[0041], fig. 2, 4 EP          2263843     A1 | |
| JP     2017-209136     A | 30 November 2017 | (Family: none) | |
| JP     2001-225814     A | 21 August 2001 | (Family: none) | |
| JP     2013-226709     A | 07 November 2013 | (Family: none) | |

Form PCT/ISA/210 (patent family annex) (January 2015)

**REFERENCES CITED IN THE DESCRIPTION**

**Patent documents cited in the description**

- JP 6091373 B